Europäisches Patentamt

European Patent Office

Office européen des brevets

(19)

(11) **EP 0 577 801 B1**

(12) **EUROPÄISCHE PATENTSCHRIFT**

(45) Veröffentlichungstag und Bekanntmachung des Hinweises auf die Patenterteilung:
**09.04.1997 Patentblatt 1997/15**

(21) Anmeldenummer: **93902184.6**

(22) Anmeldetag: **14.01.1993**

(51) Int. Cl.[6]: **A61K 7/40**, A61K 7/48,
A61K 7/00

(86) Internationale Anmeldenummer:
**PCT/EP93/00059**

(87) Internationale Veröffentlichungsnummer:
**WO 93/14741 (05.08.1993 Gazette 1993/19)**

(54) **HAAR- UND KÖRPERBEHANDLUNGSMITTEL**

HAIR AND BODY TREATMENT AGENT

AGENT DE TRAITEMENT POUR SOINS CAPILLAIRES ET CORPORELS

(84) Benannte Vertragsstaaten:
**DE ES FR GB IT**

(30) Priorität: **01.02.1992 DE 4202964**

(43) Veröffentlichungstag der Anmeldung:
**12.01.1994 Patentblatt 1994/02**

(73) Patentinhaber: **Wella Aktiengesellschaft**
**64295 Darmstadt (DE)**

(72) Erfinder:
• **BIMCZOK, Rudolf**
**D-6104 Seeheim (DE)**
• **STIEHM, Thomas**
**D-6112 Gross-Zimmern (DE)**

(56) Entgegenhaltungen:
**EP-A- 0 500 946**          **WO-A-90/09738**
**FR-A- 2 496 642**

• **CHEMICAL ABSTRACTS, vol. 116, no. 7, 17. Februar 1992, Columbus, Ohio, US; abstract no. 57863b, Y. MATSUDA 'SYNERGISTIC ODORLESS FOOD PRESERVATIVIES CONTAINING HINOKITIOL AND ETHANOL AND/OR MONOGLYCERIDES'**
• **DATABASE WPI Week 9045, Derwent Publications Ltd., London, GB; AN 90-338465 & JP,2 243 607 (ARUSOA SOGO KEKYUS) 27. September 1990**
• **PATENT ABSTRACTS OF JAPAN vol. 13, no. 339 (C-624)1989 & JP,A,11 017 820 (NOEVIR CO LTD)**
• **CHEMICAL ABSTRACTS, vol. 116, no. 6, 10. Februar 1992, Columbus, Ohio, US; abstract no. 46323z, T. NAGAHATA 'ORAL BACTERICIDAL COMPOSITIONS CONTAINING QUATERNARY AMMONIUM SALTS AND HINOKITIOL'**
• **ZENTRALBLATT FÜR BAKTERIOLOGIE MIKROBIOLOGIE UND HYGIENE Bd. 172, Nr. 6 , 1981 Seiten 508 - 519 D. REHN ET AL. 'ZUR ANTIMIKROBIELLEN WIRKSAMKEIT SUBSTITUIERTER AROMATISCHER ALDEHYDE'**
• **DATABASE WPI Week 8841, Derwent Publications Ltd., London, GB; AN 88-289041 & JP,A,63 211 217 (KANEBO KK)**

**Beschreibung**

Die Erfindung betrifft ein wäßriges Haar- und Körperbehandlungsmittel mit einem Gehalt an einer Kombination aus (a) Hinokitiol und (b) Heliotropin.

Haar- und Körperbehandlungsmittel enthalten in der Regel einen Konservierungsstoff, welcher die Mittel vor dem Befall durch Mikroorganismen wirksam schützen soll, sowie Duftstoffe, die den Mitteln einen angenehmen Duft verleihen sollen.

Für den Einsatz in Haar- und Körperbehandlungsmitteln werden an einen Konservierungsstoff gegensätzliche Anforderungen gestellt. So sollte er einerseits in physiologischer und dermatologischer Hinsicht gut verträglich sein und andererseits über eine gute keimhemmende oder sogar keimtötende Wirkung verfügen. Beide Anforderungen sind meist nur schwer miteinander vereinbar.

Als übliche in Haar- und Körperbehandlungsmitteln verwendete Konservierungsstoffe seien beispielsweise Formaldehyd (a), 5-Brom-5-nitro-1,3-dioxan (b), p-Hydroxybenzoesäureester (c), 2,4,4'-Trichlor-2'-hydroxy-diphenylether (d), 5-Chlor-2-methyl-3-isothiazolon (e), 2-Methyl-3-isothiazolon (f) und 2-Brom-2-nitropropan-1,3-diol (g) genannt.

In letzter Zeit sind einige Konservierungsstoffe, wie beispielsweise Formaldehyd und 2-Methyl-3-isothiazolon, in den Verdacht geraten, nicht genügend verträglich zu sein. Von konservierend wirkenden Aldehyden und Phenolen ist bekannt, daß diese mit Proteinen reagieren oder mit diesen in denaturierender Weise in Wechselwirkung treten. Bei Aldehyden besteht zudem die Gefahr der Sensibilisierung.

Ein weiteres Risiko, welches gegen eine Verwendung derartiger Konservierungsmittel spricht, ist die Gefahr der Nitrosaminbildung. Bei Konservierungsstoffen, welche Nitrogruppen enthalten ((b) + (g)) ist dieses Risiko besonders groß, wenn sie zusammen mit anderen stickstoffhaltigen Komponenten des kosmetischen Mittels eingesetzt werden.

Aus diesem Grunde besteht bei immer mehr Verbrauchern der Wunsch nach Haar- und Körperbehandlungsmitteln, welche die vorgenannten Konservierungsmittel nicht enthalten und zusätzlich einen angenehmen und stabilen Geruch besitzen.

Es wurde schon mehrfach versucht, die bisher üblichen Konservierungsstoffe durch neue, physiologisch besser verträgliche Stoffe zu ersetzen, jedoch konnte keine der neuen Verbindungen allen gestellten Anforderungen gerecht werden.

So wurde in der eigenen EP-OS 0 346 582 vorgeschlagen, kosmetische Alkohole, wie zum Beispiel Ethanol, Propanol oder Isopropanol, für die Konservierung von anionischen Haarpflegeemulsionen einzusetzen. Nachteilig ist jedoch, daß für eine ausreichende Konservierung mindestens 9 Gewichtsprozent Alkohol erforderlich sind, wodurch die Stabilität der Emulsion beinträchtigt werden kann.

Ebenfalls ist es aus der Literatur, beispielsweise B. Ziolkowsky "Konservierung kosmetischer Mittel" in Seifen-Öl-Fette-Wachse 112 (10), Seiten 355 bis 364 bekannt, Hinokitiol zur Konservierung von kosmetischen Mitteln zu verwenden, wobei jedoch angemerkt wird, daß Hinokitiol auch bei höheren Konzentrationen für die Konservierung von Öl-in-Wasser-Emulsionen nicht geeignet ist. In Anbetracht des hohen Preises von Hinokitiol erscheint zudem die Verwendung von höheren Einsatzmengen auch aus ökonomischen Gründen nicht sinnvoll zu sein.

Heliotropin, welches einer der Hauptbestandteile des Fliederduftes ist, ist ebenfalls für die Konservierung kosmetischer Mittel wenig geeignet, da für eine ausreichende Konservierung mehr als 0,5 Gewichtsprozent Heliotropin erforderlich sind, was jedoch zu einer erheblichen Beeinträchtigung des Geruchs des kosmetischen Mittels führt.

Es bestand daher die Aufgabe, ein Haar- und Körperpflegemittel mit einem Gehalt an einer Wirkstoffkombination zur Verfügung zu stellen, welche eine hervorragende keimhemmende beziehungsweise keimtötende Wirkung besitzt, eine gute physiologische Verträglichkeit aufweist und darüber hinaus alleine oder in Kombination mit weiteren Duftstoffen als Duftkomponente eingesetzt werden kann.

Hierzu wurde nun überraschenderweise gefunden, daß ein wäßriges Haar- und Körperbehandlungsmittel, welches eine Kombination von für sich alleine nicht ausreichend konservierend wirkenden Mengen an Hinokitiol und Heliotropin enthält, die gestellte Aufgabe in hervorragender Weise löst.

Gegenstand der vorliegenden Erfindung ist daher ein wäßriges Haar- und Körperbehandlungsmittel, welches dadurch gekennzeichnet ist, daß es eine Kombination aus (A) 0,001 bis 0,1 Gewichtsprozent Hinokitiol und (B) 0,01 bis 0,1 Gewichtsprozent Heliotropin 5 enthält.

Die bevorzugte Einsatzmenge an Hinokitiol beträgt in dem erfindungsgemäßen Haar- und Körperbehandlungsmittel 0,01 bis 0,1 Gewichtsprozent, während das Heliotropin vorzugsweise in einer Menge von 0,02 bis 0,1 Gewichtsprozent verwendet wird.

Besonders bevorzugt sind solche Haar- und Körperbehandlungsmittel in denen die Menge an Hinokitiol und Heliotropin jeweils 0,05 Gewichtsprozent beträgt.

Das erfindungsgemäße Haar- und Körperbehandlungsmittel liegt vorzugsweise in Form einer wäßrigen Lösung, einer Öl-in-Wasser-Emulsion oder einer Wasser-in-Öl-Emulsion, beispielsweise als Lotion, Gel, Creme, Aerosolspray oder Aerosolschaum, vor und besitzt einen pH-Wert von 2 bis 9, vorzugsweise 5 bis 9.

Das neue Haar- und Körperbehandlungsmittel kann beispielsweise vorliegen als Haar- und/oder Körperreinigungsmittel, als Tönungsshampoo, als Frisiercreme, Frisierlotion, Fönlotion, Mittel zur Festigung der Frisur, Waschlotion, Haarkur, Haarspülung, Mittel zum Auftragen vor oder nach der Haarfärbung und als kosmetisches Mittel zur Pflege oder zum Schutz der Haut. Beispiele für ein derartiges Mittel zur Pflege oder zum Schutz der Haut sind Gesichtswässer, Rasierwässer, Feuchthaltecremes, Coldcremes, Körperlotionen, Sonnenschutzmittel oder auch Make-up-Präparate.

Vorzugsweise enthält das erfindungsgemäße Haar- und Körperbehandlungsmittel 0,1 bis 50 Gewichtsprozent, insbesondere 10 bis 25 Gewichtsprozent, mindestens eines anionischen, kationischen, nicht-ionischen oder amphoteren Tensides.

Von den für das Haar- und Körperbehandlungsmittel geeigneten Tensiden seien beispielsweise die folgenden genannt:

a) Die anionischen oberflächenaktiven Agenzien, wie beispielsweise die Alkali-, Erdalkali-, Ammonium- oder Alkanolaminsalze von Alkansulfonaten, Alkylsulfonaten und Alkylethersulfaten, die $C_{12}$ bis $C_{18}$-Alkyl- und insbesondere $C_{12}$ bis $C_{14}$-Alkyl-Sulfatnatriumsalze oder -Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, Alkylpyridiniumsalze, beispielsweise Lauryl- oder Cetylpyridiniumsalze, beispielsweise Lauryl- oder Cetylpyridiniumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agenzien wie beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamidostearat-betain.

Selbstverständlich kann das erfindungsgemäße Mittel neben den genannten Bestandteilen auch weitere übliche kosmetische Zusätze, beispielsweise Parfümöle in einer Menge von etwa 0,01 bis 5,0 Gewichtsprozent, Trübungsmittel, wie zum Beispiel Ethylenglykoldistearat, in einer Menge von etwa 0,1 bis 5,0 Gewichtsprozent, Perlglanzmittel, wie zum Beispiel ein Gemisch aus Fettsäuremonoalkylolamid und Ethylenglykoldistearat, in einer Menge von etwa 1,0 bis 10,0 Gewichtsprozent, Verdickungsmittel, wie beispielsweise Kokosfettsäurediethanolamid, in einer Menge von etwa 0,5 bis 10,0 Gewichtsprozent, Verdünnungsmittel, wie zum Beispiel 1,2-Propylenglykol, niedere aliphatische Alkohole oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa 0,1 bis 1,0 Gewichtsprozent, Lösungsvermittler, wie zum Beispiel ethoxyliertes, gegebenenfalls hydriertes Rizinusöl, in einer Menge von etwa 0,1 bis 1,0 Gewichtsprozent, weiterhin haar- und hautpflegende Zusätze, wie zum Beispiel Fettsäureester, Fettalkohole, Fettsäureglyceride, ethoxylierte oder propoxylierte gesättigte Fettalkohole, natürliche, modifizierte natürliche oder synthetische Polymere, wie zum Beispiel Cellulosederivate, kationische Cellulosederivate, Chitosan, kationische Chitinderivate oder Polymerisate von Acrylsäure und/oder deren Derivaten, Pflegestoffe, wie zum Beispiel Lanolinderivate, Cholesterin und Pantothensäure in einer Menge von etwa 0,1 bis 10 Gewichtsprozent, außerdem physiologisch verträgliche anorganische Salze, wie zum Beispiel Natriumchlorid, sowie ferner Feuchthaltemittel, Farbstoffe, Lichtschutzmittel, Antioxidantien, Komplexbildner, Kräuterextrakte und Antischuppenwirkstoffe enthalten.

Weitere übliche, für derartige Mittel bekannte Bestandteile, welche in dem neuen Mittel enthalten sein können, sind beispielsweise bei H. Janistyn, "Handbuch der Kosmetika und Riechstoffe", 3. Band, (1973), Seiten 228 - 284 und 442 - 462, K. Schrader, "Grundlagen und Rezepturen der Kosmetika", (1979), Seiten 375 - 401 und 445 - 455 sowie G. A. Nowak, "Die kosmetischen Präparate", (1984), Seiten 452 - 512, beschrieben.

Die Zubereitung der erfindungsgemäßen Haar- und Körperbehandlungsmittel erfolgt unter Anwendung klassischer Verfahrensweisen. Beispielsweise kann man zur Bildung einer Emulsion oder einer Creme eine wäßrige Phase, die das Hinokitiol und das Heliotropin sowie gegebenenfalls weitere Bestandteile oder Hilfsstoffe gelöst enthält, und eine ölige Phase emulgieren. Für die ölige Phase kann man verschiedenartige Verbindungen verwenden, beispielsweise Paraffinöl, Vaselinöl, Süßmandelöl, Avocadoöl, Olivenöl, Fettsäureester, wie zum Beispiel Sorbitanoleat, Octylstearat, Cetylstearylisononanoat, Ethylpalmitat und Isopropylpalmitat, Alkylmyristate, wie zum Beispiel Propylmyristat, Butylmyristat und Cetylmyristat, oder Fettsäureglyceride, wie zum Beispiel Glycerinmonostearat oder Caprin-/Caprylsäure-Triglyceride. Man kann sie auch mit Fettsäurealkoholen, beispielsweise Cetyl- oder Stearylalkohol, oder Wachsen, beispielsweise Bienenwachs oder Wollwachs, versetzen.

Wenn das Mittel zur Haar- und Körperbehandlung in Form eines Aerosolpräparates - beispielsweise eines Aerosolsprays oder Aerosolschaumes - vorliegt, welches aus einem Druckbehälter entnommen wird, so enthält es zusätzlich 10 bis 60 Gewichtsprozent eines Treibmittels. Als Treibmittel können beispielsweise Chlorfluoralkane, leichtflüchtige Kohlenwasserstoffe, wie zum Beispiel n-Butan oder n-Propan, Dimethylether, Kohlendioxid, Distickstoffmonoxid, Stickstoff, Methylenchlorid oder 1,1,1-Trichlorethan Verwendung finden.

Die erfindungsgemäßen Haar- und Körperbehandlungsmittel mit einem Gehalt an der hier beschriebenen synergetische Kombination enthaltenden Haar- und Körperbehandlungsmittel sind hervorragend gegen Keimbefall geschützt und besitzen zudem einen angenehmen Geruch.

Dies ist insbesondere deswegen überraschend, weil Hinokitiol und Heliotropin jeweils für sich alleine verwendet, wenn überhaupt, erst bei wesentlich höheren Einsatzkonzentrationen eine konservierende Wirkung besitzen.

Die Kombination aus

(A) 0,001 bis 0,1 Gewichtsprozent Hinokitiol und

(B) 0,01 bis 0,1 Gewichtsprozent Heliotropin

kann deshalb zum Schutz von Haar- und Körperbehandlungsmitteln gegen den Befall durch Mikroorganismen eingesetzt werden.

Ebenfalls kann die Kombination aus

(A) 0,001 bis 0,1 Gewichtsprozent Hinokitiol und

(B) 0,01 bis 0,1 Gewichtsprozent Heliotropin,

gegebenenfalls unter Zusatz weiterer Duftstoffe,
als Duftkomponente für Haar- und Körperbehandlungsmitteln verwendet werden.

Die nachfolgenden Beispiele sollen den Gegenstand der Erfindung näher erläutern, ohne diesen auf diese Beispiele zu beschränken.

**Beispiele**

Beispiel 1 Haar- und Körperreinigungsmittel

| | |
|---|---|
| 10,00 g | Natriumlaurylethersulfat |
| 3,50 g | Natriumlaurylethercarboxylat mit 4 Ethylenoxideinheiten im Molekül |
| 3,00 g | Lauryldimethylammoniumhydroxypropylkollagenhydrolysat |
| 0,60 g | Polyethylenglykol-(3)-distearat |
| 0,50 g | Parfümöl |
| 0,02 g | Hinokitiol |
| 0,02 g | Heliotropin |
| 82,36 g | Wasser |
| 100,00 g | |

Das Haar- und Körperreinigungsmittel ist hervorragend gegen Keimbefall geschützt.

Beispiel 2 Hautpflegelotion

| | |
|---|---|
| 10,5 g | Octylstearat |
| 9,5 g | Cetylstearylisononanoat |
| 2,0 g | Sorbitanoleat |
| 2,0 g | hydriertes Rizinusöl, ethoxyliert mit 7 Mol Ethylenoxid |
| 0,5 g | Magnesiumsulfat |
| 0,1 g | Hinokitiol |

0,1 g        Heliotropin

75,3 g      Wasser

100,0 g

Beispiel 3 Hautpflegelotion

7,00 g      Paraffinöl
7,00 g      Octyldodecanol
4,00 g      Trilaurin
3,50 g      Cetylstearylalkohol
2,00 g      Caprylsäure/Caprinsäure-Triglycerid
1,20 g      Lanolin (Adeps lanae)
0,90 g      Polyglyceryl-2-sesquiisostearat
0,80 g      tris-(Tetraoxyethylencetylstearylether)-phosphat
0,40 g      Polyacrylsäure (Carbopol® 940 der Firma BF Goodrich, USA)
0,05 g      Heliotropin
0,03 g      Hinokitiol
73,12 g     Wasser

100,00 g

Beispiel 4 Vergleichsversuche

Zur Überprüfung des synergistischen Effektes der in den erfindungsgemäßen Mitteln verwendeten konservierenden Kombination wurden verschiedene Öl-in-Wasser- und Wasser-in-Öl-Emulsionen bezüglich ihrer Keimanfälligkeit miteinander verglichen.

Die Güte der Konservierung der einzelnen Emulsionen wurde hierbei durch den in der Literatur "The United States Pharmacopeia", 21. Auflage, (1985), Seite 1151 beschriebenen Konservierungsbelastungstest festgestellt. Hierbei wurden die Haarbehandlungsmittel mit den Mikroorganismen Candida albicans (ATCC Nr. 10231), Aspergillus niger (ATCC Nr. 16404). Escherichia coli (ATCC Nr. 8739), Pseudomonas aeruginosa (ATCC Nr. 9027) und Staphylocuccus aureus (ATCC NR. 6538) verunreinigt und die Entwicklung der Keimzahl über einen Zeitraum von 28 Tagen überwacht.

Die Ergebnisse dieser Konservierungsbelastungstests sind in den nachfolgenden Tabellen zusammengefaßt.

Die folgenden Emulsionen wurden miteinander verglichen:

Wasser-in-Öl-Emulsionen (Tabelle 1)

(I) Mittel gemäß Beispiel 2

(II) Mittel gemäß Beispiel 2 in dem das Heliotropin mengengleich durch Wasser ersetzt wurde.

(III) Mittel gemäß Beispiel 2 in dem die erfindungsgemäße Kombination aus 0,1 Gewichtsprozent Hinokitiol und 0,1 Gewichtsprozent Heliotropin durch 0,3 Gewichtsprozent Heliotropin ersetzt wurde.

Öl-in-Wasser-Emulsionen (Tabelle 2)

(IX) Mittel gemäß Beispiel 3

(X) Mittel gemäß Beispiel 3 in dem die erfindungsgemäße Kombination aus 0,03 Gewichtsprozent Hinokitiol und 0,05 Gewichtsprozent Heliotropin durch 0,5 Gewichtsprozent Hinokitiol ersetzt wurde.

Wie die nachfolgenden Tabellen zeigen, ist bei Verwendung der erfindungsgemäßen Kombination eine gute bis sehr gute Konservierung von Öl-in-Wasser- und Wasser-in-Öl-Emulsionen möglich, während bei Verwendung von einzelnen Komponenten dieser Kombination keine zufriedenstellende Konservierung erreicht werden kann.

Tabelle 1

| Wasser-in-Öl-Emulsion | Nr. Beurteilung der Konservierung (+ = gute bis sehr gute Konservierung - = nicht ausreichende Konservierung |
|---|---|
| (I) | + |
| (II) | - |
| (III) | - |

Tabelle 2

| Öl-in-Wasser-Emulsion | Nr. Beurteilung der Konservierung (+ = gute bis sehr gute Konservierung - = nicht ausreichende Konservierung |
|---|---|
| (IX) | + |
| (X) | - |

Beispiel 5 Selbstkonservierend wirkende Parfümöl-Zusammensetzung

| | |
|---|---|
| 20,3 g | Jasmin absolue (1 %-ige wäßrige Lösung) |
| 15,9 g | Heliotropin |
| 14,3 g | Hydroxycitronellal |
| 14,3 g | Sandelholzbase (10 %-ige wäßrige Lösung) |
| 14,3 g | Lignofix (10 %-ige wäßrige Lösung) |
| 12,8 g | Tuberose abs. synth. (10 %-ige wäßrige Lösung) |
| 4,8 g | Hinokitiol |
| 3,3 g | Bergamottöl (10 %-ige wäßrige Lösung) |
| 100,0 g | |

Bereits bei Zusatz von 0,6 Gewichtsprozent dieser Parfümölzusammensetzung (entsprechend einer Kombination aus 0,029 Gewichtsprozent Hinokitiol und 0,095 Gewichtsprozent Heliotropin) wird ein hervorragender Schutz von Wasser-in-Öl-Emulsionen gegen Keimbefall erzielt und gleichzeitig der Emulsion ein angenehmer Duft verliehen.

Alle Prozentangaben stellen, soweit nicht anders angegeben, Gewichtsprozente dar.

**Patentansprüche**

1. Wäßriges Haar- und Körperbehandlungsmittel, dadurch gekennzeichnet, daß es eine Kombination aus

   (A) 0,001 bis 0,1 Gewichtsprozent Hinokitiol und
   (B) 0,01 bis 0,1 Gewichtsprozent Heliotropin enthält.

2. Mittel gemäß Anspruch 1, dadurch gekennzeichnet, daß es eine Kombination aus

   (A) 0,05 Gewichtsprozent Hinokitiol und
   (B) 0,05 Gewichtsprozent Heliotropin enthält.

3. Mittel gemäß einem der Ansprüche 1 oder 2, dadurch gekennzeichnet, daß es in Form einer wäßrigen Lösung, einer Öl-in-Wasser-Emulsion oder einer Wasser-in-Öl-Emulsion vorliegt.

4. Mittel gemäß einem der Ansprüche 1 bis 3, dadurch gekennzeichnet, daß es einen pH-Wert von 2 bis 9 aufweist.

**5.** Mittel gemäß einem der Ansprüche 1 bis 4, dadurch gekennzeichnet, daß es zusätzlich 0,1 bis 50 Gewichtsprozent mindestens eines anionischen, kationischen, nicht-ionischen oder amphoteren Tensides enthält.

**6.** Mittel gemäß einem der Ansprüche 1 bis 5, dadurch gekennzeichnet, daß es ein Haar- und/oder Körperreinigungsmittel, ein Tönungsshampoo, eine Frisiercreme, eine Frisierlotion, eine Fönlotion, ein Mittel zur Festigung der Frisur, eine Waschlotion, eine Haarkur, eine Haarspülung, ein Mittel zum Auftragen vor oder nach der Haarfärbung oder ein kosmetisches Mittel zur Pflege oder zum Schutz der Haut ist.

## Claims

**1.** Aqueous hair and body treatment agent, characterised in that it contains a combination of

(A) 0.001 to 0.1 weight % of hinokitiol and
(B) 0.01 to 0.1 weight % of heliotropin.

**2.** Agent according to Claim 1,
characterised in that it contains a combination of

(A) 0.05 weight % of hinokitiol and
(B) 0.05 weight % of heliotropin.

**3.** Agent according to any one of Claim 1 or 2, characterised in that it is in the form of an aqueous solution, an oil-in-water emulsion or a water-in-oil emulsion.

**4.** Agent according to any one of Claims 1 to 3, characterised in that it has a pH of from 2 to 9.

**5.** Agent according to any one of Claims 1 to 4, characterised in that it additionally contains 0.1 to 50 weight % of at least one anionic, cationic, non-ionic or amphoteric surfactant.

**6.** Agent according to any one of Claims 1 to 5, characterised in that it is a hair and/or body cleansing agent, a tinting shampoo, a shaving cream, a shaving lotion, a blow-drying lotion, a hair-setting agent, a washing lotion, a hair treatment, a hair rinse, an agent to be applied before or after hair dyeing, or a cosmetic agent for the care or protection of the skin.

## Revendications

**1.** Produit de traitement des cheveux et du corps, caractérisé en ce qu'il contient une combinaison de :

(A) 0,001 à 0,1 pourcent en poids d'hinokitiol et

(B) 0,01 à 0,1 pourcent en poids d'héliotropine.

**2.** Produit selon la revendication 1 , caractérisé en ce qu'il contient une combinaison de :

(A) 0,05 pourcent en poids d'hinokitiol et

(B) 0,05 pourcent en poids d'héliotropine.

**3.** Produit selon l'une des revendications 1 ou 2 , caractérisé en ce qu'il se présente sous forme de d'une solution aqueuse, d'une émulsion huile dans l'eau, ou d'une émulsion eau dans l'huile.

**4.** Produit selon l'une des revendications 1 à 3 , caractérisé en ce qu'il présente un pH compris dans la plage allant de 2 à 9.

**5.** Produit selon l'une des revendications 1 à 4 , caractérisé en ce qu'il contient en plus de 0,1 à 50 pourcent en poids d'au moins un tensio-actif anionique, cationique, non ionique ou amphotère.

**6.** Produit sel on l'une des revendications 1 à 5, caractérisé en ce qu'il est un produit de nettoyage des cheveux et/ou du corps, un shampooing colorant, une crème de coiffure, une lotion de coiffure, une lotion de séchage des cheveux, un produit de fixation de la coiffure, une lotion de lavage, un traitement capilaire, un produit de rinçage des cheveux, un produit à appliquer avant ou après la coloration des cheveux ou un produit cosmétique pour les soins ou la protection de la peau.